# EUROPEAN PATENT APPLICATION

(11) **EP 4 305 986 A1**
(43) Date of publication of application: **17.01.2024**
(21) Application number: 23182170.3
(22) Date of filing: 28.06.2023
(51) Int. Cl.: A24F 40/51, A24F 40/53, A61M 15/00

(54) **AEROSOL DOSE TESTING DEVICE, AEROSOL-GENERATING DEVICE, AND HEATING CONTROL METHOD THEREOF**

(30) Priority: 12.07.2022 CN 202210812403
(71) Applicant: Shenzhen Smoore Technology Limited, Shenzhen, Guangdong 518102 (CN)
(72) Inventor: YANG, Wei, Shenzhen, Guangdong, 518102 (CN); XIAO, Feng, Shenzhen, Guangdong, 518102 (CN); YUAN, Huakai, Shenzhen, Guangdong, 518102 (CN)
(74) Representative: Manitz Finsterwald Patent- und Rechtsanwaltspartnerschaft mbB

(57) **Abstract**

An aerosol dose testing device, applied to an aerosol-generating device which includes a flow channel structure for flowing an aerosol. The testing device includes a light emitting module, arranged on an inner wall of the flow channel structure for providing an emitting light into a flow channel; and a photosensitive module, arranged on the inner wall of the flow channel structure corresponding to a projection path of a scattering light, for receiving the scattering light and outputting a detection signal based on an output end. The scattering light includes a light formed by scattering of the emitting light projected onto the aerosol in the flow channel structure, and the detection signal is used for determining a dose of the aerosol.

## Description

### TECHNICAL FIELD

This application relates to the field of vaporization technologies, and in particular, to an aerosol dose testing device, an aerosol-generating device, and a heating control method thereof.

### BACKGROUND

An inhalation apparatus with a vaporization function can vaporize substances such as drugs and the substances are inhaled by a user in the form of an aerosol, thereby achieving a medical effect that benefits health.

However, the applicant has found that, during an implementation process, excessive inhalation can have a negative impact, so a dose of an inhaled substance needs to be accurately detected. However, an inhalation volume detection solution in the traditional techniques has a problem of low accuracy.

### SUMMARY

In an embodiment, the present invention provides an aerosol dose testing device, applied to an aerosol-generating device which includes a flow channel structure configured to flow an aerosol. The testing device includes a light emitting module, arranged on an inner wall of the flow channel structure and configured to provide an emitting light into a flow channel; and a photosensitive module, arranged on the inner wall of the flow channel structure corresponding to a projection path of a scattering light, and configured to receive the scattering light and output a detection signal based on an output end, wherein the scattering light comprises a light formed by scattering of the emitting light projected onto the aerosol in the flow channel structure, and the detection signal is used for determining a dose of the aerosol.

### BRIEF DESCRIPTION OF THE DRAWINGS

Subject matter of the present disclosure will be described in even greater detail below based on the exemplary figures. All features described and/or illustrated herein can be used alone or combined in different combinations. The features and advantages of various embodiments will become apparent by reading the following detailed description with reference to the attached drawings, which illustrate the following:
FIG. 1 is a schematic structural diagram of an aerosol dose testing device according to an embodiment;
FIG. 2 is a schematic structural diagram of an aerosol dose testing device according to an embodiment;
FIG. 3 is a schematic diagram in which an emitting light passes through an aerosol mass in a flow channel and is projected onto an extinction structure to eliminate a reflected light in the flow channel according to an embodiment;
FIG. 4 is a schematic structural diagram of an aerosol dose testing device according to an embodiment;
FIG. 5 is a schematic waveform diagram of pulse signals detected under different flow rates of an aerosol according to an embodiment;
FIG. 6 is a schematic structural diagram of an aerosol dose testing device according to an embodiment;
FIG. 7 is a schematic structural diagram of an aerosol-generating device according to an embodiment;
FIG. 8 is a schematic flowchart of a method for testing a dose of an aerosol according to an embodiment;
FIG. 9 is a schematic flowchart of a heating control method of an aerosol-generating device according to an embodiment;
FIG. 10 is a schematic partial flowchart of a heating control method according to an embodiment;
FIG. 11 is a schematic structural diagram of an apparatus for testing a dose of an aerosol according to an embodiment;
FIG. 12 is a schematic structural diagram of a heating control apparatus of an aerosol-generating device according to an embodiment; and
FIG. 13 is a schematic structural diagram of an electronic device according to an embodiment.

### DETAILED DESCRIPTION

In an embodiment, the present invention provides an aerosol dose testing device, an aerosol-generating device, and a heating control method thereof that can accurately detect a dose of an aerosol for the foregoing technical problem.

According to a first aspect, an aerosol dose testing device is provided, applied to an aerosol-generating device, where the aerosol-generating device is provided with a flow channel structure for flowing of an aerosol. The testing device includes: a light emitting module, arranged on the inner wall of the flow channel structure and configured to provide an emitting light into a flow channel; and a photosensitive module, arranged on the inner wall of the flow channel structure corresponding to a projection path of a scattering light, and further configured to receive the scattering light and output a detection signal through an output end. The scattering light is a light formed by scattering of the emitting light projected onto the aerosol in the flow channel, and the detection signal is used for determining a dose of the aerosol.

In an embodiment, the aerosol dose testing device further includes: an extinction structure, arranged on the wall of the flow channel structure corresponding to a projection direction of the emitting light and configured to eliminate the received emitting light.

In an embodiment, the aerosol dose testing device further includes: an amplification circuit, where an input end of the amplification circuit is connected to the output end of the photosensitive module.

In an embodiment, the aerosol dose testing device further includes a filter circuit. An input end of the filter circuit is connected to the photosensitive module, and an output end of the filter circuit is connected to the input end of the amplification circuit.

In an embodiment, the photosensitive module includes at least one of a photoelectric sensor or a solar battery.

In an embodiment, the light emitting module includes at least one of a laser or a light emitting diode.

According to a second aspect, an aerosol-generating device is further provided, including: a vaporizer, provided with a flow channel structure for flowing of an aerosol and configured to heat an aerosol-generating substrate to generate the aerosol; and the foregoing aerosol dose testing device.

In an embodiment, the vaporizer includes: a heating component, configured to heat the aerosol-generating substrate; and a controller, connected to the heating component, the light emitting module, and the photosensitive module, configured to control the heating component to perform heating, and further configured to determine the dose of the aerosol according to the detection signal.

In an embodiment, the controller is further configured to control the heating component to stop heating when the determined dose of the aerosol reaches a preset upper limit value.

In an embodiment, the aerosol-generating device further includes: an inhalation detection module, arranged at a vapor outlet of the flow channel structure and configured to detect a duration of each inhalation action. The controller is connected to the inhalation detection module, further configured to calculate a dose of an inhaled aerosol according to the duration of each inhalation action and the detection signal obtained within the duration, and configured to control the heating component to stop heating when the dose of the inhaled aerosol reaches the preset upper limit value.

According to a third aspect, a method for testing a dose of an aerosol is further provided, applied to the foregoing aerosol dose testing device, and the method including: controlling, during a process of generating the aerosol by the aerosol-generating device, the light emitting module to provide the emitting light into the flow channel; and obtaining the detection signal output by the photosensitive module; and determining the dose of the aerosol according to the detection signal.

According to a fourth aspect, a heating control method of the aerosol-generating device is further provided, applied to the foregoing aerosol-generating device, and the method including: controlling the vaporizer to heat the aerosol-generating substrate to generate the aerosol; obtaining the detection signal during a process of generating the aerosol; determining the dose of the aerosol according to the detection signal; and controlling the vaporizer to stop heating when the determined dose of the aerosol reaches a preset upper limit value.

In an embodiment, the heating control method further includes: obtaining a duration of each inhalation action detected by an inhalation detection module, where the inhalation detection module is arranged at a vapor outlet of the flow channel structure; and the determining the dose of the aerosol according to the detection signal includes: calculating a dose of an inhaled aerosol according to the duration of each inhalation action and the detection signal obtained within the duration.

According to a fifth aspect, an aerosol dose testing apparatus is further provided, applied to the foregoing aerosol dose testing device, and the apparatus including: a light emitting control module, configured to control, during a process of generating the aerosol by the aerosol-generating device, the light emitting module to provide the emitting light into the flow channel; a first detection signal obtaining module, configured to obtain the detection signal output by the photosensitive module; and a first dose determining module, configured to determine the dose of the aerosol according to the detection signal.

According to a sixth aspect, a heating control apparatus of the aerosol-generating device is further provided, applied to the foregoing aerosol-generating device, and the apparatus including: a heating control module, configured to control the vaporizer to heat the aerosol-generating substrate to generate the aerosol; a second detection signal obtaining module, configured to obtain the detection signal during a process of generating the aerosol; a second dose determining module, configured to determine the dose of the aerosol according to the detection signal; and a stop heating control module, configured to control the vaporizer to stop heating when the determined dose of the aerosol reaches a preset upper limit value.

According to a seventh aspect, an electronic device is provided, including a memory and a processor, the memory storing a computer program, where the processor, when executing the computer program, implements the steps of the foregoing method.

According to an eighth aspect, a computer-readable storage medium is provided, storing a computer program, where when the computer program is executed by a processor, the steps of the foregoing method are implemented.

For the foregoing aerosol dose testing device, the aerosol-generating device, and the heating control method thereof, the light emitting module and the photosensitive module are arranged in the flow channel structure of the aerosol-generating device. The light emitting module may be arranged on the inner wall of the flow channel structure to provide the emitting light into the flow channel. The photosensitive module is arranged on the projection path of the scattering light formed by scattering the emitting light onto the aerosol in the flow channel, and is configured to receive the scattering light and perform photoelectric conversion to generate the detection signal. The detection signal is used for indicating the intensity of the scattering light, the intensity of the scattering light is related to the density of the aerosol, and the detection signal is also related to the flow rate of the aerosol. Based on this, the detection signal may be used to determine the dose of the aerosol, thereby achieving aerosol dose testing. The detection signal provides key and reliable data for not excessive inhalation of the aerosol. Compared to conventional techniques that only measure the inhalation amount of the aerosol from the time dimension, the solution provided in embodiments of this application has high detection accuracy. In addition, the implementation of the entire testing is simple, fast, and low cost. The aerosol-generating device equipped with the aerosol dose testing device can determine the dose of the generated aerosol, determine the level of the dose of the inhaled aerosol, and control the aerosol-generating device to stop heating the aerosol-generating substrate when the dose reaches the upper limit value for healthy inhalation.

For ease of understanding this application, this application is described more comprehensively below with reference to the related accompanying drawings. The accompanying drawings show embodiments of this application. However, this application may be implemented in many different forms, and is not limited to the embodiments described in this specification. On the contrary, the embodiments are provided to make the disclosed content of this application more comprehensive.

Unless otherwise defined, meanings of all technical and scientific terms used in this specification are the same as those usually understood by a person skilled in the art to which this application belongs. In this specification, terms used in this specification of this application are merely intended to describe objectives of the specific embodiments, but are not intended to limit this application.

It may be understood that terms "first", "second" and the like used in this application may be used to describe various elements in this specification, but the elements are not limited by the terms. The terms are merely used for distinguishing a first element from another element.

It should be noted that, when an element is considered to be "connected" to another element, the element may be directly connected to the another element, or connected to the another element through a central element. In addition, "connected" in the following embodiments is to be interpreted as "electrically connected", "communicatively connected", and the like if electrical signals or data is transferred between the connected objects.

When used herein, the singular forms "a", "an" and "the" may also include the plural form, unless otherwise clearly indicated. It should be further understood that the terms "include/comprises" or "have", etc. specify the presence of stated features, integers, steps, operations, components, parts, or combinations thereof, but do not preclude the possibility of the presence or addition of one or more other features, integers, steps, operations, components, parts, or combinations thereof. In addition, the term "and/or" used in this specification includes any and all combinations of related listed items.

An inhalation apparatus with a vaporization function can vaporize substances such as solid substrates of leafy plants, liquid substrates such as essential oils, or drugs, etc., and these substances are inhaled by a user in the form of an aerosol, thereby achieving a medical effect that benefits health. Inhaled substances act on the human body, and excessive inhalation may have a negative impact on the human body, so a dose of an inhaled substance needs to be accurately detected. Current technical means mainly calculate the dose of the inhaled substance through inhalation time. However, the accuracy of a dose of an aerosol detected by this detection method is poor.

After a research, the inventor found that the reason for this problem lies in the fact that in conventional technologies, aerosol inhalation detection is mainly considered a time dimension. However, in addition to an output time of the aerosol, the inhalation dose of aerosol is also affected by dimensional data such as the density of vaporized substances, inhalation strength (flow rate of vaporized substances), and the like.

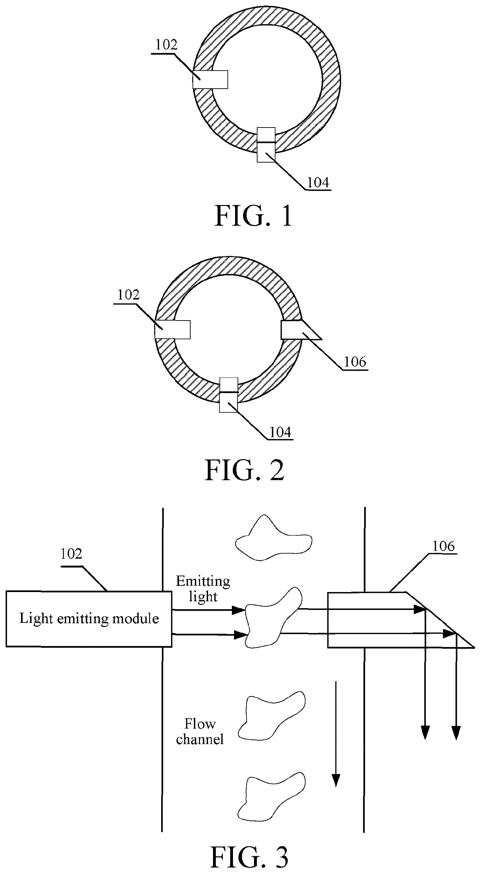

Based on the foregoing reason, this application provides an aerosol dose testing device, applied to an aerosol-generating device, where the aerosol-generating device is provided with a flow channel structure for flowing of an aerosol. As shown in FIG. 1, the testing device includes a light emitting module 102 and a photosensitive module 104. The light emitting module 102 is arranged on the inner wall of the flow channel structure. As those skilled in the art can understand, the light emitting module 102 being arranged on the inner wall of the flow channel structure may be implemented by being at least partially embedded in the inner wall or being attached to the surface of the inner wall. This application does not limit the specific installation implementation method of the light emitting module 102. An installation position of the light emitting module 102 is based on the principle of ensuring that the light emitting module 102 can provide an emitting light into the flow channel during operation. When an aerosol is flowing in the flow channel, the emitting light projected by the light emitting module 102 strikes the aerosol, and scatters through the aerosol. There is a certain relationship between the density of the aerosol and the intensity of the scattering light, so the density of the aerosol can be determined by detecting the intensity of the scattering light. The photosensitive module 104 is arranged on the projection path of the scattering light to receive the scattering light formed by passing through the aerosol in the flow channel, and convert a scattering light signal into a detection signal for output. During flowing of the aerosol in the flow channel, there is flow noise similar to a mass object, and there is a certain statistical inheritance in the flow direction. The faster the flow rate of the aerosol, the shorter the flight time of the mass object when passing through the beam. Based on this correspondence, the detection signal may be used to determine the flow rate of the aerosol in the flow channel. The detection signal may indicate the density of the aerosol and the flow rate of the aerosol, and a combination of the density of the aerosol, the flow rate of the aerosol, and the generation time of the aerosol may determine the dose of the aerosol, thereby affecting the judgment of the amount of the aerosol inhalation. The photosensitive module 104 is mounted on the inner wall of the flow channel structure for a stable installation. The scattering light is a light formed by the scattering of the emitting light projected onto the aerosol in the flow channel. Certainly, when the dose of the aerosol is determined, for an aerosol-generating device with a stable flow rate, the flow rate may be regarded as a known definite value in this case. The density of the aerosol is calculated only through the detection signal, and the accuracy of the dose of the aerosol that is determined based on the density of the aerosol and the inhalation time is relatively high. Similarly, for an aerosol-generating device with good consistency in the density of the generated aerosol, the density of the aerosol may be regarded as a definite value in this case, or only the flow rate of the aerosol is calculated based on the detection signal, and the dose of the aerosol is determined based on the flow rate and the inhalation time.

It could be understood that the light emitting module 102 refers to a device or a module such as a light emitting diode, a laser, or the like that is capable of emitting an optical signal. The photosensitive module 104 refers to a module that can receive light and convert an optical signal into an electrical signal. For example, the photosensitive module 104 may be a photosensitive transistor (such as a photosensitive diode, a photosensitive triode, and the like.), a solar battery, and the like.

The intensity of the scattering light is related to the density of the aerosol, and the detection signal is also related to the flow rate of the aerosol. Based on this, the detection signal may be used to determine the dose of the aerosol, thereby achieving aerosol dose testing. The detection signal provides key and reliable data for not excessive inhalation of the aerosol. Compared to conventional techniques that only measure the inhalation amount of the aerosol from the time dimension, the solution provided in embodiments of this application has higher detection accuracy. In addition, the implementation of the entire testing is simple, fast, and low cost.

Considering that the emitting light projected by the light emitting module 102 passes through or does not pass through the aerosol and is projected on the inner wall of the opposite flow channel structure, a reflected light may be formed by reflection from the inner wall of the flow channel structure, and the reflected light may also be projected on the photosensitive module 104, which causes interference to the detection signal output by the photosensitive module 104. Based on this, in order to further improve the testing accuracy, in an embodiment, as shown in FIG. 2 and FIG. 3, the aerosol dose testing device further includes: an extinction structure 106, arranged on the wall of the flow channel structure corresponding to a projection direction of the emitting light and configured to eliminate the received emitting light.

The extinction structure 106 is a structure for eliminating the received emitting light to reduce interference of the reflected light to the photosensitive module 104. The extinction structure 106 may include, but is not limited to, a multiple reflection structure, a conical structure, or the like. The elimination of the emitting light may be achieved by changing the projection path of the emitting light, thereby suppressing the reflection of the emitting light, and projecting the received emitting light on a structure outside the flow channel. For example, the extinction structure 106 may project the emitting light on the aerosol-generating substrate through projection, so that the light energy is used for auxiliary heating to achieve secondary utilization of residual heat.

To better illustrate the implementation process of the aerosol dose testing device provided in this embodiment of this application, FIG. 2 is taken as an example, a group of a light emitting module 102 and an extinction structure 106 may be arranged on the cross-section of the flow channel of the aerosol. The light emitted by the light emitting module 102 passes through the aerosol and then is incident to the extinction structure 106. After a light source is incident to the aerosol, scattering light is generated. A photosensitive module 104 may be arranged at a certain angle to the main optical axis of the emitting light. The photosensitive element is configured to receive the scattering light and output a detection signal that can indicate the intensity of the scattering light, and then the detection signal is used to calculate the corresponding density of the aerosol. If the density of the aerosol is known, the dose of the inhaled aerosol may further be calculated.

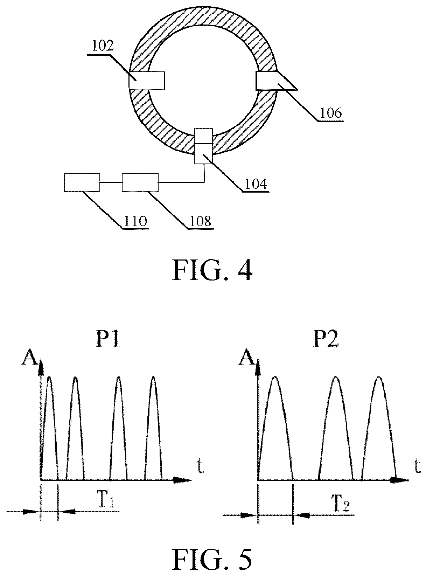

As described in the foregoing embodiment, during flowing of a fluid, there is some flow noise inside similar to a mass substance, and there is a certain statistical inheritance in the flow direction. After the mass substance enters a radiation region of the emitting light, a scattering light corresponding to the mass substance is generated. When the mass substance leaves the beam, the scattering light generated by the mass substance disappears. Therefore, after the mass substance passes through the beam, the photosensitive module 104 generates a scattering light pulse signal that can indicate a change of the scattering light (the detection signal is a pulse signal, and the pulse change condition corresponds to the occurrence and disappearance of the scattering light). Considering that the pulse signal is generally weak, in order to improve the accuracy of calculation, in an embodiment, as shown in FIG. 4, the aerosol dose testing device further includes: an amplification circuit 110. An input end of the amplification circuit is connected to an output end of the photosensitive module 104. The detection signal (scattering light pulse signal) output by the photosensitive module 104 is processed and converted into a voltage pulse signal by the amplification circuit 110, for example, the amplification circuit 110 may include an operational amplification circuit 111, and the time width of the pulse signal is the flight time for passing through the emitting light of the mass substance. The time width of the pulse is inversely correlated with the flow rate of the air flow. Based on this inverse correlation, the flow rate of the aerosol may be calculated based on the time width of the pulse of an amplified detection signal.

As shown in FIG. 5, a pulse width T1 of an amplified voltage pulse signal P1 of a detection signal J1 is less than a pulse width T2 of a voltage pulse signal P2 corresponding to an other detection signal J2, that is, the flow rate of the air flow tested by the voltage pulse signal P1 is greater than the flow rate of the air flow tested by the voltage pulse signal P2. That is, the flow rate of the aerosol during the sampling period of the detection signal J1 is greater than the flow rate during the sampling period of the detection signal J2. In the figure, A represents amplitude and t represents time.

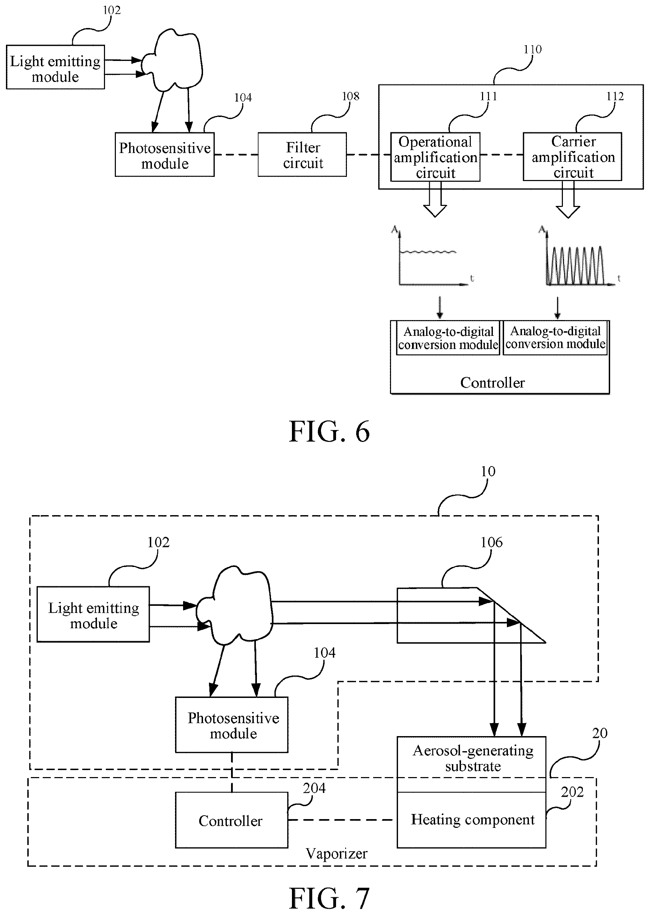

To improve the accuracy of the test results and avoid the noise being amplified, in an embodiment, as shown in FIG. 4 and FIG. 6, the aerosol dose testing device further includes: a filter circuit 108. An input end of the filter circuit is connected to the photosensitive module 104, and an output end of the filter circuit is connected to the input end of the amplification circuit 110. The filter circuit 108 filters the detection signal output by the photosensitive module 104 and then outputs the detection signal to the amplification circuit 110. In this case, the amplification circuit 110 amplifies the filtered detection signal, which can avoid a great calculation error of the dose caused by noise amplification, thereby improving the accuracy of aerosol dose testing.

As described in the foregoing embodiment, the testing device provided in this embodiment of this application can adopt a unified optical structure for a light scattering optical system for detecting the density of the aerosol and a light pulse system for detecting the flow rate of the air flow, that is, the structures of the light emitting module 102 and the photosensitive module 104 are adopted. An optical system of the light emitting module 102, the photosensitive module 104, and the extinction structure 106 may further be used together. In this case, the emitting light is incident in the extinction structure 106 after passing through the aerosol, and scattering light is generated when the emitting light is incident to the aerosol. The scattering light is converted into an electrical detection signal through the photosensitive module 104, and the detection signal is output through the operational amplification processing. The output signal may be transmitted to the controller 204 (for example, it may be a microprocessor) in the aerosol-generating device, and is calculated by the controller 204 to obtain the density of the aerosol. In addition, the electrical detection signal of the photosensitive module 104 may also be filtered and amplified for identifying the pulse signal generated by the mass substance. The pulse signal may be output to the controller 204, and be processed and calculated by the controller 204 to obtain the flow rate of the air flow.

In an embodiment, as shown in FIG. 6, the amplification circuit 110 may include an operational amplification circuit 111 and a carrier amplification circuit 112. The input end of the operational amplification circuit 111 is connected to the output end of the photosensitive module 104, and the input end of the carrier amplification circuit 112 is connected to the output end of the operational amplification circuit 111. Certainly, the output signals of the operational amplification circuit 111 and the carrier amplification circuit 112 may be output and transmitted to the controller 204 of the aerosol-generating device for calculating the dose of the aerosol. The detection signal that is operationally amplified may be used for calculating the density of the aerosol. For the detection signal that is carrier amplified, the pulse waveform changes more significantly, which facilitates the determining of the flow rate of the aerosol according to the pulse width, and improves the calculation accuracy of the flow rate of the aerosol.

The density of the aerosol and the flow rate of the aerosol are detected in real time through the optical system. The integration of the density and the flow rate within a specific time period is the total dose of the aerosol within the time period. The specific time period may be a defined treatment period and the like. The defined treatment period refers to a single treatment duration when the aerosol-generating device is used for performing a vaporization treatment. In order to avoid excessive or insufficient inhalation of the aerosol during a single treatment period, it is necessary to test the total dose of the aerosol within the specific time period.

In an embodiment, the photosensitive module 104 includes at least one of a photoelectric sensor or a solar battery. The wavelength of the emitting light may be visible light, infrared light, or ultraviolet light. The photoelectric sensor may include a photoelectric transistor, a photosensitive resistor, and the like, and the photoelectric transistor may also include a photo diode, a photoelectric triode, and the like. Any device that is capable of performing photoelectric conversion may be used as the photosensitive module 104. When the photoelectric sensor is used to achieve the output of the detection signal, a small size is suitable for a small aerosol-generating device, such as an integrated aerosol-generating device. When the solar battery is used, the scattering light is converted into the detection signal, at the same time, the detection signal may be reused for energy storage, and when the aerosol-generating device heats the aerosol-generating substrate, electrical energy is provided, which is beneficial to improving the endurance of the aerosol-generating device. The aerosol dose testing device, when mounted on the aerosol-generating device with a smaller size, may be implemented by using a highly integrated solar chip.

In an embodiment, the light emitting module 102 includes at least one of a laser or a light emitting diode. The laser has the characteristics of high brightness and high directionality, which can ensure the intensity of the scattering light received by the photosensitive module 104, thereby improving reliability of the detection and avoiding the problem of undetected scattering light caused by too weak emitting light. In addition, based on the high directionality of the laser, the installation position of the photosensitive module 104 is convenient to be determined, which can effectively avoid the error of the detection signal caused by the emitting light that is emitted by the light emitting module 102 directly projecting on the photosensitive module 104. As shown in FIG. 7, the emitting light projected by the laser on the extinction structure 106 may be projected on the aerosol-generating substrate through the extinction structure 106 for laser heating.

The light emitting module 102 may also use a device such as a light emitting diode, which is small in size, low in cost, and low in energy consumption.

An embodiment of this application further provides an aerosol-generating device, as shown in FIG. 7, including: a vaporizer 20, provided with a flow channel structure for flowing of an aerosol and configured to heat an aerosol-generating substrate to generate the aerosol; and the foregoing aerosol dose testing device 10.

The structure of the vaporizer 20 is not limited herein, and may be integral or split. When the structure is split, the vaporizer may include a first part and a second part. The first part may include a heating component 202, and is provided with an accommodating cavity. The accommodating cavity is configured to accommodate the aerosol-generating substrate. The second part may include a battery and a controller 204. The battery is electrically connected to the controller 204. During operation, the battery supplies power to the controller 204, and the controller 204 controls the heating component 202 to heat the aerosol-generating substrate in the accommodating cavity to generate the aerosol. Certainly, the split vaporizer 20 is not limited to the structural forms of the first part and the second part herein, the heating component 202 may also be arranged in the second part. When the first part and the second part are used together, the heating component 202 may heat the aerosol-generating substrate.

The aerosol-generating device equipped with the aerosol dose testing device 10 can determine the dose of the aerosol by using the detection signal output by the photosensitive module 104, thereby further determining the level of the dose of the inhaled aerosol, and control the aerosol-generating device to stop heating the aerosol-generating substrate when the dose reaches the upper limit value for healthy inhalation.

The vaporizer 20 may have a certain computing power, be able to receive the detection signal, and perform calculations on the detection signal to obtain the dose of the aerosol. The dose of the aerosol in this embodiment of this application may be based on the dose output from the vapor outlet of the aerosol-generating device. For example, for a headworn respirator, because all the aerosol generated by the aerosol-generating device enter the human body based on the closed environment of the respirator during a breathing process, in this case, the dose of the aerosol inhaled by the human body can be indicated by the amount of the aerosol actually output by the aerosol-generating device. For an open aerosol-generating device, for example, in a situation in which it is necessary to match the inhalation action of the user to achieve aerosol intake, the dose of the aerosol inhaled by the human body refers to the amount of the aerosol inhaled by the human body from the aerosol-generating device, which differs from the dose of the aerosol output by the aerosol-generating device. The implementation process of calculating the dose of the aerosol by the vaporizer 20 may be achieved by calculating the flow rate of the aerosol and the density of the aerosol according to the detection signal, and performing integral calculation within the duration for heating and generating the aerosol by the vaporizer 20 to calculate the dose of the aerosol within the duration.

In an embodiment, the cross-section of the flow channel that is close to the vapor outlet is circular. The light emitting module 102 is arranged on the inner wall of the flow channel structure, and a junction between the line that connects the light emitting module 102 and the center of the circle and the flow channel structure is provided with the foregoing extinction structure 106. On the circumference of the cross-section, a photosensitive module 104 is arranged at a position between the light emitting module 102 and the extinction structure 106. For example, as shown in FIG. 1, FIG. 2, and FIG. 4, the light emitting module 102, the photosensitive module 104, and the extinction structure 106 are sequentially arranged at equal intervals along the circumferential direction of the flow channel structure.

In an embodiment, the vaporizer 20 includes a heating component 202 and a controller 204. The heating component 202 is configured to heat the aerosol-generating substrate. The controller 204 is connected to the heating component 202, the light emitting module 102, and the photosensitive module 104. The controller 204 has functions of control and calculation, configured to control the heating component 202 to perform heating, and further control the heating component 202 to stop heating. The controller 204 may also determine the dose of the aerosol according to the detection signal, reference may be made to the description in the foregoing embodiment, and description is not repeated herein again.

In an embodiment, the controller 204 is further configured to control the heating component 202 to stop heating when the determined dose of the aerosol reaches a preset upper limit value. Considering the health hazards caused by excessive dose of the inhaled aerosol, the controller 204 of the aerosol-generating device provided in this embodiment of this application can calculate that during the process of continuously heating and outputting the aerosol by the aerosol-generating device, the dose of the inhaled aerosol has reached the preset upper limit value. At this time, the heating component 202 is controlled to stop heating, which avoids causing damage to human health. The preset upper limit value is a value that does not affect human health and is allowed to inhale the dose of the aerosol. This value can be configured, and the corresponding preset upper limit value may vary for different aerosol-generating substrates. For example, for different drugs, the upper limit values of the drugs intake during a single treatment often differ, which makes it necessary to configure different preset upper limit values for different aerosol-generating substrates.

Therefore, in an implementation, the aerosol-generating device further includes an input apparatus. The input apparatus is connected to the controller 204, and the input apparatus is configured to input the preset upper limit value. Based on the structure that can input the configuration, a single set of aerosol-generating device may be applied to a plurality of scenarios.

Certainly, for an aerosol-generating device of which the application scenario is determined, the preset upper limit value thereof may be configured before leaving the factory.

In an embodiment, the aerosol-generating device further includes: an inhalation detection module (not shown in the figures), arranged at the vapor outlet of the flow channel structure. The user may inhale the aerosol at the vapor outlet. During the inhalation of the aerosol by the user, the inhalation detection module detects the duration of each inhalation action by the user, because for the user, only when the inhalation action is performed can the aerosol be effectively inhaled. Based on this, the controller 204 is connected to the inhalation detection module, so that the controller 204 can calculate a more accurate dose of the aerosol inhaled by the user according to the duration of each aspiration action and the detection signal obtained during the duration. The interference of the dose of the aerosol output by the aerosol-generating device into the atmosphere may be removed when the user does not perform the inhalation action. Then, based on each inhalation action of the user, the controller 204 performs a cumulative calculation of the dose of the aerosol, and controls the heating component 202 to stop heating when the dose of the inhaled aerosol reaches the preset upper limit value. Based on this control, adverse effects on human health caused by inhalation of the excessive aerosol by the user can be avoided. In addition, the problem of poor therapeutic effects caused by inhalation of the excessive aerosol can be avoided in the scenario in which the aerosol-generating substrate is a drug.

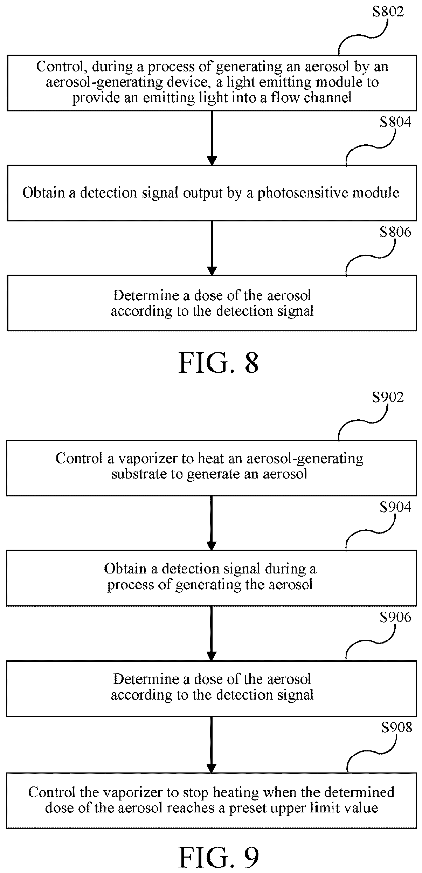

The phenomenon of light scattering occurs when the aerosol is irradiated by the incident light. The intensity of the scattering light is related to the density of the aerosol, and the density of the aerosol can be detected by detecting the intensity of the scattering light. Based on the same concept, this application further provides a method for testing a dose of an aerosol, applied to the foregoing aerosol dose testing device. As shown in FIG. 8, the method includes the following steps.

In a step S802, a light emitting module is controlled, during a process of generating an aerosol by an aerosol-generating device, to provide an emitting light into a flow channel.

In a step S804, a detection signal output by a photosensitive module is obtained.

In a step S806, a dose of the aerosol is determined according to the detection signal.

For definitions of the light emitting module, the detection signal, and the like, reference may be made to the descriptions in the foregoing device embodiments, and description is not repeated herein. During a process of generating the aerosol by the aerosol-generating device, the light emitting module is controlled to provide the emitting light into the flow channel, which may save energy. Certainly, the light emitting module may operate as soon as the aerosol-generating device is powered on. As long as it is ensured that when the aerosol flows in the flow channel, the light emitting module can provide the emitting light. The emitting light is scattered on the aerosol in the flow channel, and the generated scattering light is projected on the photosensitive module. The photosensitive module performs photoelectric conversion to output the detection signal. Referring to the description in the foregoing device embodiment, there is a correlation between the detection signal and the dose of the aerosol, so the dose of the aerosol may be determined according to the detection signal, thereby achieving aerosol dose testing.

Based on the same concept, an embodiment of this application further provides a heating control method of an aerosol-generating device, applied to the foregoing aerosol-generating device. As shown in FIG. 9, the method includes the following steps.

In a step S902, a vaporizer is controlled to heat an aerosol-generating substrate to generate an aerosol. The executing body that controls a vaporizer to heat an aerosol-generating substrate to generate an aerosol may be a separate controller or a controller inside the vaporizer.

In a step S904, a detection signal during a process of generating the aerosol is obtained. The detection signal during the process of generating the aerosol may indicate the output of the aerosol.

In a step S906, a dose of the aerosol is determined according to the detection signal. The implementation of determining the dose of the aerosol may be referred to the description in the foregoing embodiments.

In a step S908, the vaporizer is controlled to stop heating when the determined dose of the aerosol reaches a preset upper limit value. By obtaining the detection signal and calculating the dose of the aerosol based on the detection signal during the process of heating the aerosol-generating substrate, the heating of the vaporizer can be immediately stopped as soon as the dose reaches the preset upper limit value, which avoids the continued generation of the aerosol for the user to inhale, thereby avoiding the impact on the health of the user.

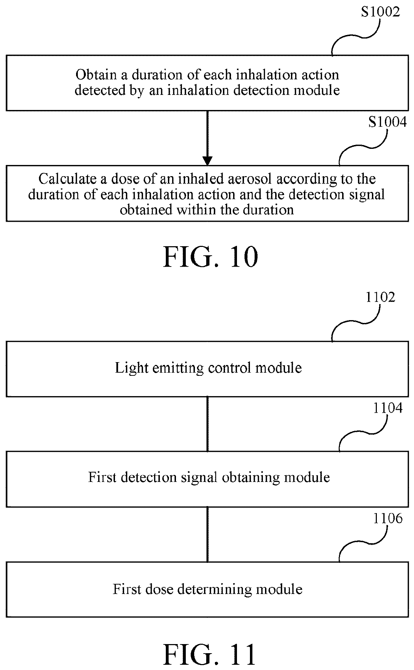

In an embodiment, as shown in FIG. 10, the heating control method further includes the following steps.

In a step S1002, a duration of each inhalation action detected by an inhalation detection module is obtained, where the inhalation detection module is arranged at a vapor outlet of the flow channel structure.

The determining the dose of the aerosol according to the detection signal includes a step S1004.

In the step S1004, a dose of an inhaled aerosol is calculated according to the duration of each inhalation action and the detection signal obtained within the duration.

Based on the description in the foregoing aerosol-generating device embodiment, it can be learned that by obtaining the duration of the inhalation action and the detection signal during each inhalation action, the dose of the inhaled aerosol can be calculated more accurately. Based on the comparison of the dose of the aerosol with the preset upper limit value, excessive inhalation of the aerosol can be avoided. In addition, stopping heating in advance due to large deviation in calculation of the dose of the aerosol can be avoided, so that insufficient inhalation is avoided.

It is to be understood that, although the steps in the flowchart are sequentially shown according to indication of an arrow, the steps are not necessarily sequentially performed according to a sequence indicated by the arrow. Unless otherwise explicitly specified in this specification, execution of the steps is not strictly limited, and the steps may be performed in other sequences. In addition, at least some steps in the figure may include a plurality of steps or a plurality of stages. The steps or the stages are not necessarily performed at the same moment, but may be performed at different moments. The steps or the stages are not necessarily performed in sequence, but may be performed in turn or alternately with another step or at least some of steps or stages of the another step.

Based on the same invention concept, an embodiment of this application further provides an apparatus for testing a dose of an aerosol for implementing the foregoing method for testing a dose of an aerosol. The implementation solution for solving the problem provided by this apparatus is similar to the implementation solution recorded in the foregoing method. Therefore, for the specific limitations in one or more embodiments of the apparatus for testing a dose of an aerosol provided below, reference may be made to the foregoing limitations for the method for testing a dose of an aerosol, and the description is not repeated herein again.

In an embodiment, an apparatus for testing a dose of an aerosol is provided, applied to the foregoing aerosol dose testing device. As shown in FIG. 11, the apparatus includes:

a light emitting control module 1102, configured to control, during a process of generating the aerosol by the aerosol-generating device, the light emitting module to provide the emitting light into the flow channel;
a first detection signal obtaining module 1104, configured to obtain the detection signal output by the photosensitive module; and
a first dose determining module 1106, configured to determine the dose of the aerosol according to the detection signal.

Specifically, the light emitting control module controls the light emitting module to provide the emitting light into the flow channel during the generation of the aerosol by the aerosol-generating device, and then the first detection signal obtaining module obtains the detection signal output by the photosensitive module and sends the detection signal to the first dose determining module, and the first dose determining module determines the dose of the aerosol according to the detection signal.

For a specific limitation on the apparatus for testing a dose of an aerosol, reference may be made to the limitation on the foregoing method for testing a dose of an aerosol. Details are not described herein again. The modules in the foregoing apparatus for testing a dose of an aerosol may be implemented entirely or partially by software, hardware, or a combination thereof. The foregoing modules may be built in or independent of a processor of an electronic device in a hardware form, or may be stored in a memory of the electronic device in a software form, so that the processor invokes and performs an operation corresponding to each of the foregoing modules. It should be noted that, in the embodiments of this application, the division of the modules is merely an example, and is merely division of logical functions. During actual implementation, there may be another division manner.

Based on the same invention concept, an embodiment of this application further provides a heating control apparatus of the aerosol-generating device for implementing the foregoing heating control method of the aerosol-generating device. The implementation solution for solving the problem provided by this apparatus is similar to the implementation solution recorded in the foregoing method. Therefore, for the specific limitations in one or more embodiments of the heating control apparatus of the aerosol-generating device provided below, reference may be made to the foregoing limitations for the heating control method of the aerosol-generating device, and the description is not repeated herein again.

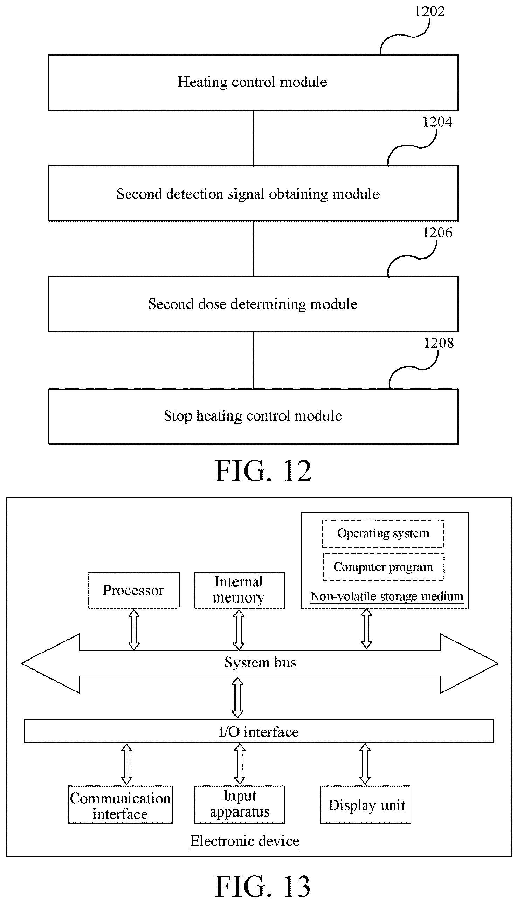

In an embodiment, a heating control apparatus of the aerosol-generating device is provided, applied to the foregoing aerosol-generating device. As shown in FIG. 12, the apparatus includes:
a heating control module 1202, configured to control the vaporizer to heat the aerosol-generating substrate to generate the aerosol; a second detection signal obtaining module 1204, configured to obtain the detection signal during a process of generating the aerosol; a second dose determining module 1206, configured to determine the dose of the aerosol according to the detection signal; and
a stop heating control module 1208, configured to control the vaporizer to stop heating when the determined dose of the aerosol reaches a preset upper limit value.

Specifically, the heating control module controls the vaporizer to heat the aerosol-generating substrate to generate the aerosol, and then the second detection signal obtaining module obtains the detection signal during the process of generating the aerosol. The second dose determining module determines the dose of the aerosol according to the detection signal sent by the second detection signal obtaining module. When the determined dose of the aerosol reaches the preset upper limit value, the stop heating control module controls the vaporizer to stop heating to avoid excessive inhalation of the aerosol.

For a specific limitation on the heating control apparatus of the aerosol-generating device, reference may be made to the limitation on the foregoing heating control method of the aerosol-generating device. Details are not described herein again. The modules in the foregoing heating control apparatus of the aerosol-generating device may be implemented entirely or partially by software, hardware, or a combination thereof. The foregoing modules may be built in or independent of a processor of an electronic device in a hardware form, or may be stored in a memory of the electronic device in a software form, so that the processor invokes and performs an operation corresponding to each of the foregoing modules. It should be noted that, in the embodiments of this application, the division of the modules is merely an example, and is merely division of logical functions. During actual implementation, there may be another division manner.

In an embodiment, an electronic device is provided. The electronic device may be a controller or a control chip, and an internal structure diagram thereof may be shown in FIG. 13. The electronic device includes a processor, a memory, a communication interface, a display screen, and an input apparatus that are connected by using a system bus. The processor of the electronic device is configured to provide computing and control capabilities. The memory of the electronic device includes a non-transitory storage medium and an internal memory. The non-transitory storage medium stores an operating system and a computer program. The internal memory provides an environment for running of the operating system and the computer program in the non-transitory storage medium. The communication interface of the electronic device is configured to communicate with an external terminal in a wired or a wireless manner, and the wireless manner can be implemented by using WIFI, an operator network, NFC, or other technologies. The computer program is executed by the processor to implement a method for testing a dose of an aerosol and/or the foregoing heating control method. The memory may store the foregoing preset upper limit value for reading. The display screen of the electronic device may be a liquid crystal display screen or an electronic ink display screen, configured to remind when the dose of the inhaled aerosol exceeds the preset upper limit value.

A person skilled in the art may understand that in the structure shown in FIG. 13, only a block diagram of a partial structure related to a solution in this application is shown, and FIG. 13 does not constitute a limitation to the electronic device to which the solution in this application is applied. Specifically, the electronic device may include more components or fewer components than those shown in FIG. 13, or some components may be combined, or a different component deployment may be used.

In an embodiment, an electronic device is provided, including a memory and a processor, the memory storing a computer program, when executed by the processor, causing the processor to perform the following steps:

controlling, during a process of generating the aerosol by the aerosol-generating device, the light emitting module to provide the emitting light into the flow channel;
obtaining the detection signal output by the photosensitive module; and
determining the dose of the aerosol according to the detection signal.

In an embodiment, an electronic device is provided, including a memory and a processor, the memory storing a computer program, when executed by the processor, causing the processor to perform the following steps:
controlling the vaporizer to heat the aerosol-generating substrate to generate the aerosol;
obtaining the detection signal during a process of generating the aerosol; determining the dose of the aerosol according to the detection signal; and
controlling the vaporizer to stop heating when the determined dose of the aerosol reaches a preset upper limit value.

In an embodiment, the processor, when executing the computer program, further implements the following steps:
obtaining a duration of each inhalation action detected by an inhalation detection module, where the inhalation detection module is arranged at a vapor outlet of the flow channel structure; and
calculating a dose of an inhaled aerosol according to the duration of each inhalation action and the detection signal obtained within the duration.

In an embodiment, a computer-readable storage medium is provided, storing a computer program, the computer program, when executed by a processor, causing the processor to implement the following steps:
controlling, during a process of generating the aerosol by the aerosol-generating device, the light emitting module to provide the emitting light into the flow channel;
obtaining the detection signal output by the photosensitive module; and
determining the dose of the aerosol according to the detection signal.

In an embodiment, a computer-readable storage medium is provided, storing a computer program, the computer program, when executed by a processor, causing the processor to implement the following steps:
obtaining a duration of each inhalation action detected by an inhalation detection module, where the inhalation detection module is arranged at a vapor outlet of the flow channel structure; and
calculating a dose of an inhaled aerosol according to the duration of each inhalation action and the detection signal obtained within the duration.

In an embodiment, the computer program, when executed by a processor, further causes the processor to implement the following steps:
obtaining a duration of each inhalation action detected by an inhalation detection module, where the inhalation detection module is arranged at a vapor outlet of the flow channel structure; and
calculating a dose of an inhaled aerosol according to the duration of each inhalation action and the detection signal obtained within the duration.

In an embodiment, a computer program product is further provided, storing a computer program, the computer program, when executed by a processor, implementing some or all of the steps in the foregoing method embodiments, and implementing the corresponding beneficial effects.

A person of ordinary skill in the art may understand that all or some of the procedures of the methods in the foregoing embodiments may be implemented by a computer program instructing relevant hardware. The computer program may be stored in a non- transitory computer-readable storage medium. When the program is executed, the procedures of the foregoing method embodiments may be performed. Any reference to a memory, a storage, a database, or another medium used in the embodiments provided in this application may include at least one of a non-transitory memory and a volatile memory. The non- transitory memory may include a read-only memory (ROM), a magnetic tape, a floppy disk, a flash memory, an optical memory, and the like. The volatile memory may include a random access memory (RAM) or an external cache. For the purpose of description instead of limitation, the RAM is available in a plurality of forms, such as a static RAM (SRAM) or a dynamic RAM (DRAM), etc.

## Claims

1. An aerosol dose testing device (10), applied to an aerosol-generating device, wherein the aerosol-generating device is provided with a flow channel structure for flowing of an aerosol, and the testing device comprising:
a light emitting module (102), arranged on an inner wall of the flow channel structure and configured to provide an emitting light into a flow channel; and
a photosensitive module (104), arranged on the inner wall of the flow channel structure corresponding to a projection path of a scattering light, and further configured to receive the scattering light and output a detection signal through an output end, wherein
the scattering light is a light formed by scattering of the emitting light projected onto the aerosol in the flow channel structure, and the detection signal is used for determining a dose of the aerosol.

2. The aerosol dose testing device (10) according to claim 1, further comprising: an extinction structure (106), arranged on a wall of the flow channel structure corresponding to a projection direction of the emitting light and configured to eliminate the received emitting light.

3. The aerosol dose testing device (10) according to claim 1 or claim 2, further comprising:
an amplification circuit (110),
wherein an input end of the amplification circuit (110) is connected to the output end of the photosensitive module (104).

4. The aerosol dose testing device (10) according to claim 3, further comprising:
a filter circuit (108),
wherein an input end of the filter circuit (108) is connected to the photosensitive module (104), and
wherein an output end of the filter circuit (108) is connected to the input end of the amplification circuit (110).

5. The aerosol dose testing device (10) according to any one of claims 1 to 4, wherein the photosensitive module (104) comprises at least one of a photoelectric sensor or a solar battery.

6. The aerosol dose testing device (10) according to any one of claims 1 to 5, wherein the light emitting module (102) comprises at least one of a laser or a light emitting diode.

7. An aerosol-generating device, comprising:
a vaporizer (20), provided with a flow channel structure for flowing of an aerosol and configured to heat an aerosol-generating substrate to generate the aerosol; and
the aerosol dose testing device (10) according to any one of claims 1 to 6.

8. The aerosol-generating device according to claim 7, wherein the vaporizer (20) comprises:
a heating component (202), configured to heat the aerosol-generating substrate; and
a controller (204) connected to the heating component (202), the light emitting module (102), and the photosensitive module (104), the controller (204) being configured to control the heating component (202) to perform heating, and configured to determine the dose of the aerosol according to the detection signal.

9. The aerosol-generating device according to claim 8, wherein the controller (204) is further configured to control the heating component (202) to stop heating when the determined dose of the aerosol reaches a preset upper limit value.

10. The aerosol-generating device according to claim 9, further comprising:
an inhalation detection module arranged at a vapor outlet of the flow channel structure and configured to detect a duration of each inhalation action,
wherein the controller (204) is connected to the inhalation detection module and configured to calculate a dose of an inhaled aerosol according to the duration of each inhalation action and the detection signal obtained within the duration, and configured to control the heating component (202) to stop heating when the dose of the inhaled aerosol reaches the preset upper limit value.

11. A method for testing a dose of an aerosol, applied to the aerosol dose testing device of claim 1, comprising:
controlling, during a process of generating the aerosol by the aerosol-generating device, the light emitting module to provide the emitting light into the flow channel (S802);
obtaining the detection signal output by the photosensitive module (S804); and
determining the dose of the aerosol according to the detection signal(S806).

12. A heating control method of an aerosol-generating device, applied to the aerosol-generating device of claim 7, comprising:
controlling the vaporizer to heat the aerosol-generating substrate to generate the aerosol (S902);
obtaining the detection signal during a process of generating the aerosol (S904);
determining the dose of the aerosol according to the detection signal (S906); and
controlling the vaporizer to stop heating when the determined dose of the aerosol reaches a preset upper limit value (S908).

13. The heating control method according to claim 12, further comprising:
obtaining a duration of each inhalation action detected by an inhalation detection module (S1002), the inhalation detection module being arranged at a vapor outlet of the flow channel structure,
wherein the determining the dose of the aerosol according to the detection signal comprises:
calculating a dose of an inhaled aerosol according to the duration of each inhalation action and the detection signal obtained within the duration (S1004).

14. An electronic device, comprising:
a memory; and
a processor,
wherein the memory stores a computer program, and
wherein the processor, when executing the computer program, implements the steps of the method according to any one of claims 11 to 13.

15. One or more non-transitory computer-readable storage mediums having processor-executable instructions stored thereon, wherein the processor-executable instructions, when executed, facilitates the method according to any one of claims 11 to 13.
